# FASCICULE DE BREVET EUROPEEN

(11) **EP 0 848 041 B1**
(45) Date de publication et mention de la délivrance du brevet: **29.08.2001**
(21) Numéro de dépôt: 97402988.6
(22) Date de dépôt: 10.12.1997
(51) Int. Cl.: C09B 61/00, C09B 5/24, A61K 7/13, C07D 221/18, C07D 209/58

(54) **Précurseur de colorant issu de plantes du genre Medicago, sa préparation et son application à l'obtention de produits colorants**
Farbstoffvorläufer aus Pflanzen der Medicagogattung, dessen Herstellung und Anwendung in der Herstellung von Farbstoffen
Dye precursor from plants of the Medicago genus, its preparation and its use in the manufacture of dyestuffs

(30) Priorité: 11.12.1996 FR 9615212
(43) Date de publication de la demande: 17.06.1998
(73) Titulaire: L'OREAL, 75008 Paris (FR)
(72) Inventeur: Belcour-Castro, Béatrice, 37520 La Riche (FR); Hussler, Georges, 93600 Aulnay-Sous-Bois (FR); Bonnet, Anne, 75019 Paris (FR)
(74) Mandataire: Tonnellier, Jean-Claude

(56) Documents cités:
- EP-A- 0 560 683
- WO-A-82/01010
- DATABASE WPI Section Ch, Week 9411 Derwent Publications Ltd., London, GB; Class B04, AN 94-089261 XP002038421 & JP 06 040 884 A (TAIYO KAGAKU KK) , 15 février 1994
- CHEMICAL ABSTRACTS, vol. 97, no. 13, 27 septembre 1982 Columbus, Ohio, US; abstract no. 107154, CHAUBET, NICOLE ET AL: "Characterization of.beta.-galactosidase of Medicago sativa suspension-cultured cells growing on lactose. Effect of the growth substrates on the activities" XP002038420 & Z. PFLANZENPHYSIOL. (1982), 106(5), 401-7,

## Description

L'invention a pour objet un précurseur de colorant pouvant être obtenu à partir de cellules d'une plante du genre Medicago, ainsi que le procédé de préparation dudit précurseur de colorant et son application à l'obtention de produits colorants, par réaction avec une quinone.

On connaît de nombreux produits colorants dérivés de quinone, ainsi que leur application notamment à la teinture des fibres kératiniques ; voir par exemple la demande de brevet EP-A-0560683 et les documents cités dans ladite demande de brevet.

Le document JP-06 040 884, dont un abrégé est publié par la base de données WPI/DERWENT, AN 94-089 261 (1994) décrit des extraits de plantes du genre *Médicago* utilisables comme ingrédients cosmétiques à effet blanchissant.

On a maintenant découvert que des cellules de plantes du genre Medicago, cultivées *in vitro* en présence de lactose comme substrat carboné, contiennent des métabolites capables de réagir avec des quinones en donnant des produits colorants, et que ces métabolites sont absents lorsque l'on utilise le glucose comme substrat carboné.

Ce sont ces métabolites qui constituent les précurseurs de colorants de l'invention. On a découvert que ces précurseurs de colorants sont également présents dans les plantes du genre Medicago cultivées sur sol ou en conditions hydroponiques, mais à une très faible concentration.

L'invention a donc pour objet une composition contenant au moins un précurseur de colorant, caractérisée par le fait que ledit précurseur de colorant est un produit existant à l'état naturel dans les cellules d'une plante du genre Medicago, est capable de réagir en solution aqueuse avec la 1,4-naphtoquinone en donnant au moins un produit colorant, et est présent dans ladite composition à l'état au moins partiellement purifié.

L'expression "à l'état au moins partiellement purifié" signifie ici que, par rapport à son état naturel (plante ou cellules fraîches ou desséchées), le précurseur de colorant, dans la composition de l'invention, a été concentré et/ou a été débarrassé d'au moins une partie des autres constituants de la plante, et en particulier des constituants ayant une masse moléculaire supérieure à 3500.

Dans les compositions de l'invention, le précurseur de colorant est généralement présent à une concentration supérieure à la concentration dudit précurseur dans ladite plante ou dans lesdites cellules à l'état desséché.

On considère qu'une plante est à l'état desséché quand sa teneur en eau est inférieure à 10 % en poids.

L'invention concerne en particulier une composition dans laquelle ledit précurseur est présent à une concentration au moins égale à celle dudit précurseur dans une dispersion aqueuse contenant, par litre de dispersion, 1 gramme (et notamment 2 grammes), en matière sèche, d'un broyat de cellules de ladite plante, les cellules dudit broyat ayant été cultivées *in vitro* dans un milieu contenant uniquement du lactose comme source de carbone.

Les précurseurs de colorants qui sont des constituants de la composition de l'invention sont présents notamment dans les plantes, et dans les cellules cultivées *in vitro* (par exemple dans les conditions indiquées ci-dessus), des espèces suivantes : Medicago sativa, Medicago falcata, Medicago intermedia, Medicago media, Medicago varia et Medicago versicolor.

La culture *in vitro* desdites plantes peut être effectuée au départ d'un explant (par exemple un fragment de feuille ou de cotylédon).

Les milieux de culture qui conviennent à la culture *in vitro* de cellules végétales sont actuellement bien connus. Ces milieux de culture contiennent notamment des sources d'azote, des sources de carbone, des microéléments, ainsi que des vitamines à faible concentration. Les milieux de culture actuels dérivent notamment des bases théoriques définies par MURASHIGE et SKOOG lors de la mise au point du milieu de culture qui porte le nom de ces auteurs. Pour les microéléments, on utilise généralement ceux proposés par Heller : manganèse, zinc, bore, cuivre, iode, nickel, aluminium, ainsi que le molybdène, le fer et le cobalt. Comme source de carbone, on utilise généralement des sucres, en particulier le saccharose et le glucose, mais pour obtenir les précurseurs de colorants de l'invention, il convient de remplacer au moins partiellement ces sucres par d'autres sources de carbone, notamment le lactose. La détermination des sources de carbone qui conviennent peut être effectuée par de simples expériences de routine en recherchant la présence, dans les cellules cultivées, d'un précurseur de colorant tel que défini dans la présente demande. Parmi les vitamines qui favorisent la croissance des cellules en culture, on citera notamment la thiamine, et aussi l'acide nicotinique et la pyridoxine. On peut ajouter également du panthoténate de calcium, de la biotine et du méso-inositol.

Le milieu de culture peut également contenir des acides aminés, des extraits protéiques, des acides organiques, etc. On peut également ajouter au milieu de culture des régulateurs de croissance tels que l'acide naphtalène acétique (ANA), la kinétine (6-furfurylamino-purine), etc.

Les compositions de précurseur de colorant de l'invention sont notamment des solutions (c'est-à-dire pratiquement exemptes de produits solides non dissous) ou des produits solides pouvant être obtenus par exemple au départ de telles solutions par évaporation du ou des solvants. Les résidus solides obtenus par évaporation du solvant (notamment eau, alcanol inférieur et leurs mélanges) sont pratiquement totalement solubles dans un tel solvant.

L'invention a également pour objet un procédé de préparation d'une composition telle que définie précédemment.

Ce procédé est essentiellement caractérisé par le fait que l'on broie des plantes ou parties de plantes du genre Medicago, ou des cellules d'une plante du genre Medicago provenant d'une culture *in vitro,* et que l'on sépare et recueille, selon les méthodes de fractionnement usuelles, une fraction du broyat obtenu, ladite fraction étant une fraction au moins partiellement purifiée exempte de constituants de masse moléculaire supérieure à 3500. Pour séparer ladite fraction au moins partiellement purifiée, on peut opérer par exemple selon l'une au moins des méthodes suivantes :
- on procède à une filtration et recueille le filtrat,
- on procède à une décantation ou à une centrifugation, et recueille une fraction contenant le surnageant,
- ou l'on procède à une extraction avec un solvant.

Le solvant approprié pour l'extraction peut être déterminé par de simples expériences de routine. On peut utiliser notamment l'eau ou un alcanol inférieur (par exemple le méthanol ou l'éthanol). On peut ensuite évaporer le solvant pour obtenir une composition sous forme d'extrait sec : on traite un broyat, un extrait, un filtrat ou un surnageant ainsi obtenu de façon à éliminer les constituants de masse moléculaire supérieure à 3500, et en particulier supérieure à 1500.

Selon un mode de réalisation particulier du procédé de préparation de la composition de précurseur de colorant, on cultive *in vitro* des cellules d'une plante du genre Medicago, dans un milieu contenant une source de carbone appropriée, par exemple du lactose, on broie la biomasse formée, et on sépare et recueille, comme précédemment, une fraction dudit broyat contenant le précurseur de colorant à l'état au moins partiellement purifié.

Il est possible d'effectuer la culture dans un milieu contenant uniquement du lactose comme source de carbone.

La culture *in vitro* peut être effectuée à la lumière, mais elle est effectuée de préférence à l'obscurité. On opère en milieu non agité ou, de préférence, en milieu agité. La température de culture peut varier par exemple de 20 à 30°C. On recueille la biomasse au bout d'un temps de culture suffisant, qui dépend notamment du volume du fermenteur utilisé. On peut également procéder, à intervalles réguliers, à des prélèvements d'une partie de la biomasse en cas de culture en continu. Dans chaque cas, le temps de culture ou les intervalles entre deux prélèvements peuvent être déterminés par des expériences de routine.

En vue d'une utilisation des compositions de l'invention, le procédé de préparation de ces compositions comprend donc une étape de broyage de la biomasse (plantes, parties de plantes ou cellules de cultures *in vitro*) selon les méthodes usuelles. On peut ensuite purifier au moins partiellement la composition en éliminant tout ou partie des constituants autres que les précurseurs de colorants, comme indiqué précédemment. Par exemple, on peut filtrer le broyat sur tout filtre approprié, y compris sur filtre retenant les produits de dimension supérieure à 0,2 µm environ.

On peut purifier encore davantage la composition de l'invention (filtrat, surnageant ou extrait) par passage à travers-une membrane d'ultrafiltration ou de dialyse capable de retenir les constituants de masse moléculaire supérieure à un seuil prédéterminé, par exemple supérieure à 3500, ou supérieure à 1500 ou à 1000. Dans chacun de ces cas, les précurseurs de colorants se trouvent dans le filtrat ou le dialysat.

Les précurseurs de colorants de l'invention sont retenus par les membranes ayant un seuil de coupure de 500 daltons. Leur masse moléculaire est donc comprise entre 500 et 1000 daltons.

L'invention a également pour objet un procédé d'obtention d'un produit colorant, caractérisé par le fait que l'on fait réagir une quinone avec une composition de précurseur de colorant telle que définie ci-dessus et que, si désiré, on isole et/ou purifie un produit colorant issu de cette réaction.

La sélection des quinones qui conviennent peut être effectuée par de simples expériences de routine en faisant réagir la quinone considérée avec une composition contenant le précurseur de colorant défini ci-dessus. En effet, la réaction conduit à l'apparition d'une coloration ou à une modification de la coloration, la quinone considérée est utilisable dans le procédé d'obtention d'un produit colorant selon l'invention.

Selon un mode de réalisation particulier, la quinone peut être choisie notamment parmi les 1,4-benzoquinones éventuellement substituées en l'une au moins des positions 2 et 3, les 1,4-naphtoquinones éventuellement substituées en l'une au moins des positions 5, 6, 7 et 8, et la diphénoquinone.

Parmi les 1,4-benzoquinones éventuellement substituées, on citera notamment celles qui répondent à la formule (I) 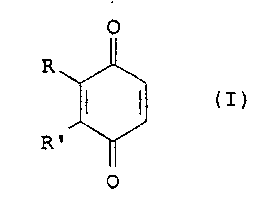 dans laquelle R et R' représentent indépendamment -H, hydroxy, alkyle en C₁-C₄, alcoxy en C₁-C₄, sulfonyle, ou un groupement hétérocyclique ou aryle, éventuellement substitué. Le groupement hétérocyclique est par exemple un groupement furanyle, pyranyle ou indolyle, éventuellement substitué, notamment substitué par un groupement alkyle ou alcoxy en C₁-C₄. Le groupement aryle est par exemple un groupement phényle éventuellement substitué, notamment substitué par au moins un alkyle ou alcoxy en C₁-C₄.

Parmi les 1,4-naphtoquinones éventuellement substituées, on citera notamment celles qui répondent à la formule (II) 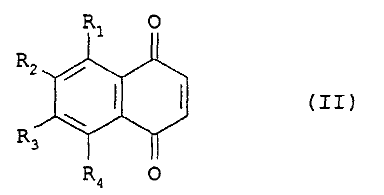 dans laquelle les substituants R₁ à R₄ sont définis, indépendamment, comme R et R' ci-dessus.

Les quinones de formules (I) et (II) sont connues ou peuvent être préparées selon des procédés connus. Certaines quinones utilisables, y compris des composés de formule (I) et (II), sont décrites par exemple dans les documents EP-376776 et "Dictionary of Natural Products on CD-ROM", CHAPMAN & HALL (London) 1996.

On peut utiliser notamment une quinone choisie parmi la 1,4-benzoquinone, la 2-méthyl-1,4-benzoquinone, la 2-(4-méthyl-2-furanyl)-1,4-benzoquinone, la 2-hydroxy-1,4-benzoquinone, la 2-phényl-1,4-benzoquinone, la 1,4-naphtoquinone, la 5-hydroxy-1,4-naphtoquinone, la 5-hydroxy-7-méthyl-1,4-naphtoquinone, la 6-méthyl-1,4-naphtoquinone et la 5,8-dihydroxy-1,4-naphtoquinone.

On peut également utiliser d'autres dérivés de quinone tels que la géogénine (ou pleurotine) et la juglorine.

La réaction du précurseur de colorant avec une quinone est effectuée de préférence en solution dans un solvant approprié tel que par exemple un alcool ou un mélange d'eau et d'alcool. L'alcool peut être notamment un alcanol inférieur tel que le méthanol. Par exemple, on ajoute la quinone sous la forme d'une solution dans un alcool à une solution aqueuse contenant le précurseur de colorant. La réaction peut être effectuée à une température de 20 à 100°C environ et en particulier de 30 à 80°C environ.

Les précurseurs de colorants de l'invention peuvent être présents dans les plantes entières cultivées sur sol ou en culture hydroponique. Ainsi, dans le cas de Medicago sativa, le filtrat obtenu après passage sur membrane de porosité 0,2 µm d'un broyat de plante entière fournit des précurseurs de colorants semblables à ceux présents dans les cellules cultivées *in vitro* en présence de lactose, comme l'a montré par exemple l'analyse par HPLC d'un tel filtrat. Toutefois, dans la plante entière, ces précurseurs de colorants ne sont le plus souvent présents qu'à de faibles concentrations, généralement plusieurs centaines de fois plus faibles que les concentrations obtenues dans les cellules de la même plante cultivées *in vitro* en présence de lactose comme seule source de carbone. Les compositions de l'invention, telles que définies ci-dessus, sont notamment celles qui contiennent les précurseurs de colorants à une concentration au moins égale à celle desdits précurseurs dans une dispersion aqueuse contenant, par litre de dispersion, 1 g (et en particulier 2 g) en matière sèche d'un broyat de cellules de la plante considérée, les cellules dudit broyat ayant été cultivées dans un milieu contenant uniquement du lactose comme source de carbone. Les concentrations minimum ainsi définies, qui peuvent être très facilement atteintes en utilisant comme matière première des cellules cultivées *in vitro* en présence d'une source de carbone telle que par exemple le lactose, sont nettement supérieures à celles qui peuvent être atteintes dans une dispersion aqueuse d'un broyat de la plante entière.

L'invention a également pour objet un produit colorant caractérisé par le fait qu'il est susceptible d'être obtenu par le procédé qui vient d'être décrit.

Parmi ces colorants, on citera notamment les composés A, B, C et D décrits ci-après dans la partie expérimentale.

Parmi les précurseurs de colorants contenus dans les compositions de l'invention, on citera donc en particulier ceux qui sont capables de réagir avec la 1,4-naphtoquinone en donnant l'un au moins desdits composés A, B, C et D.

Les produits colorants obtenus selon l'invention sont généralement solubles dans au moins un solvant usuel, par exemple dans des alcools (notamment alcanols inférieurs comme le méthanol ou l'éthanol) et dans des mélanges d'eau et d'un alcool.

Ces produits colorants peuvent être utilisés notamment dans des compositions colorantes telles que des compositions de teinture des fibres textiles, y compris la laine ; dans des compositions de teinture des cheveux ; dans des compositions de vernis à ongles, etc. De telles compositions font partie de l'invention. Ils peuvent également être utilisés pour donner une coloration distinctive à des compositions liquides telles que des compositions de nettoyage ou d'entretien des surfaces (sols, murs, vitres).

Les compositions de teinture de l'invention peuvent être préparées selon les méthodes usuelles. Par exemple, les compositions de teinture pour cheveux, contenant les produits colorants obtenus selon l'invention, comprennent au moins un solvant approprié acceptable en cosmétologie, notamment l'eau, les alcools inférieurs (par exemple en C₁-C₆), les alkylèneglycols (comme l'éthylèneglycol ou le propylèneglycol), etc.

Les produits colorants obtenus selon l'invention sont présents, dans les compositions de teinture pour cheveux, à une concentration comprise généralement de 0,01 à 10 % en poids, et en particulier de 0,05 à 5 % en poids, par rapport au poids total de la composition. Ces compositions peuvent également contenir d'autres colorants choisis parmi les colorants directs usuels, ainsi que les ingrédients usuellement présents dans des compositions de ce type, par exemple des agents tensioactifs, des agents épaississants, des agents dispersants, des agents conservateurs, des agents de gonflement des fibres kératiniques, des parfums, etc.

Les compositions de teinture de l'invention peuvent se présenter notamment sous la forme de lotions, de gels, de dispersions, d'émulsions, de mousses ou encore sous forme de compositions conditionnées pour aérosols.

Pour teindre les cheveux avec des compositions de teinture de l'invention, on applique la composition sur les cheveux et on la laisse agir, dans le cas où la composition est destinée à être rincée, pendant un temps généralement compris entre 5 et 60 minutes, et en particulier entre 10 et 30 minutes, puis on rince les cheveux. Dans le cas où la composition n'est pas destinée à être rincée, on l'applique sur les cheveux, puis on peut sécher ces derniers.

Les compositions de vernis à ongles contenant les produits colorants obtenus selon la présente invention sont préparées de manière usuelle. Outre un produit colorant obtenu selon l'invention, elles contiennent généralement, dans un solvant ou un mélange de solvants approprié (notamment des alcools tels que des alcanols inférieurs ou l'alcool benzylique, leurs acétates, ou encore l'acétone ou des glycols), un polymère filmogène (notamment nitrocellulose, éthylcellulose, etc), un plastifiant (par exemple phtalate de butyle) qui augmente notamment la souplesse du film de vernis déposé, et éventuellement d'autres colorants ou pigments, et des résines qui permettent d'obtenir un vernis brillant et ayant une bonne adhérence.

Les compositions de teinture de l'invention peuvent être également utilisées dans des procédés de teinture des fibres textiles, par exemple la laine, par imprégnation ou immersion de fibres textiles ou de tissus, avec une solution ou dispersion de produits colorants obtenus selon l'invention, éventuellement en présence d'un agent mordant, selon des méthodes connues en soi.

L'invention concerne également un nécessaire (kit) permettant de préparer une composition colorante. Ledit kit comprend, dans des conteneurs séparés dont les contenus sont à mélanger au moment de l'emploi, au moins un précurseur de colorant et au moins une quinone, ledit précurseur présentant les caractéristiques suivantes :
- il existe à l'état naturel dans les cellules d'une plante du genre Medicago,
- il est capable de réagir avec ladite quinone en donnant au moins un produit colorant.

Dans le kit de l'invention, le précurseur de colorant peut être présent en solution dans un solvant tel que ceux mentionnés ci-dessus pour la réaction avec la quinone. Le précurseur de colorant peut également être présent sous forme d'extrait sec ou de produit purifié. Dans ce dernier cas, le kit peut contenir au moins un tel solvant dans un conteneur séparé, dont le contenu est utilisé pour dissoudre les réactifs (précurseur de colorant et/ou quinone) au moment de l'utilisation. De même, la quinone peut être présente sous forme de produit pur ou sous forme de solution.

Les exemples suivants illustrent l'invention.

### EXEMPLE 1 : Culture in vitro à l'obscurité en présence de lactose

On met en culture en milieu liquide, dans des conditions aseptiques, un fragment de cotylédon de luzerne (variété Europe). Le milieu de culture, qui présente la particularité de contenir du lactose comme substrat carboné, a la composition suivante :

| *Constituants* | *Concentrations (mg.L*^{*-1*}*)* |
|---|---|
| KNO₃ | 1900,000 |
| | |
| NH₄NO₃ | 1650,000 |
| KH₂PO₄, H₂O | 170,000 |
| MgSO₄, 7H₂O | 370,000 |
| CaCl2, 2H₂O | 440,000 |
| MnSO₄, H₂O | 0,076 |
| NiCl₂, 6H₂O | 0,030 |
| AlCl₃, 6H₂O | 0,050 |
| H₃BO₃ | 1,000 |
| ZnSO₄, 7H₂O | 1,000 |
| CuSO₄, 5H₂O | 0,030 |
| KI | 0,010 |
| FeSO₄, 7H₂O | 27,800 |
| Na₂EDTA | 37,300 |
| Panthoténate de calcium | 1,000 |
| Méso-inositol | 100,000 |
| Acide nicotinique | 1,000 |
| Pyridoxine | 1,000 |
| Thiamine | 1,000 |
| Biotine | 0,010 |
| Kinétine | 0,100 |
| Acide naphtalène acétique | 0,100 |
| Lactose | 30000,0 |

Ce milieu de culture est préalablement stérilisé à l'autoclave pendant 20 minutes à 115°C.

Pour l'entretien de la culture, on récolte les cellules par filtration sur une toile de Blutex de 50 µm et on inocule du milieu de culture vierge, en fioles d'Erlenmeyer de 1 L contenant 400 mL de milieu, avec 20 g de la biomasse récoltée. La culture est maintenue à 26°C à l'abri de la lumière avec agitation à 100 rpm. L'entretien est hebdomadaire.

### EXEMPLE 2 : Extraction et caractérisation du précurseur de colorant : réaction avec la 1,4-naphtoquinone

### a) Obtention du précurseur de colorant sous la forme d'une solution filtrée

Des cellules cultivées comme à l'exemple 1 sont récoltées à la fin d'un cycle d'entretien par filtration sur toile de Blutex 50 µm. On introduit 5 g de matière fraîche ainsi récoltée dans 15 cm³ d'une solution tamponnée de pH 6,5.

La solution tampon est préparée de la façon suivante : on prépare une solution A contenant 27,8 g de NaH₂PO₄, 2H₂O dans 1 L d'eau ultrapure ; on prépare une solution B contenant 53,65 g de Na₂HPO₄, 7H₂O dans 1 L d'eau ultrapure ; on mélange 171,25 mL de solution A avec 78,75 mL de solution B et 250 mL d'eau ultrapure pour obtenir 500 mL de la solution tamponnée.

La suspension de cellules est broyée dans un homogénéiseur de Potter. Le mélange broyé est filtré sur filtre Whatmann de 2,7 µm, puis le filtrat obtenu est filtré sur membrane Millipore 0,2 µm. Les opérations de filtration sont effectuées à 4°C.

### b) Réaction avec la 1,4-naphtoquinone

A 5 cm³ du filtrat obtenu, on ajoute 50 pL d'une solution contenant 12,5 g/L de 1,4-naphtoquinone dans le méthanol. On laisse le mélange obtenu en incubation pendant 90 minutes à 30°C.

### c) Extraction de produit colorant

On ajoute ensuite une solution aqueuse d'acide chlorhydrique 1N jusqu'à pH 2,5 et on ajoute du chlorure de méthylène, à raison d'un volume pour un volume de milieu réactionnel. On agite pendant une heure avec un barreau magnétique, on centrifuge pendant 10 minutes à 10000 rpm. On sépare la phase organique et la phase aqueuse. On ajoute à la phase aqueuse de l'acétate d'éthyle (1 volume d'acétate d'éthyle pour un volume de phase aqueuse). On agite pendant une heure puis on laisse décanter et on sépare les phases organique et aqueuse.

Les phases organiques sont réunies et séchées sur sulfate de sodium anhydre. Ce dernier est ensuite éliminé par filtration. On évapore ensuite les solvants sous pression réduite à 50°C. On obtient un résidu sec.

### d) Analyse de l'extrait obtenu

Le résidu sec obtenu au stade précédent, dissous dans un mélange méthanol/chlorure de méthylène 50:50 (en volume), est analysé par CCM (chromatographie sur couche mince) de silice. On élue avec du chlorure de méthylène contenant 1 % de méthanol.

On observe la présence d'un produit A caractérisé par Rf = 0,88 et d'un produit B caractérisé par Rf = 0,85.

L'extrait est également caractérisé en HPLC, avec une colonne HPLC Lichrosorb® RP (fournisseur : MERCK). L'éluant est un mélange 60/40 (V/V) d'acétonitrile et d'eau ultrapure acidifiée jusqu'à pH 3 par l'acide phosphorique. Débit : 1 cm³ par minute.

On effectue la détection à 490 nm. Les produits A et B sont caractérisés respectivement par des temps de rétention de 22,7 et de 13,8 minutes.

L'analyse par CCM (éluant : mélange chlorure de méthylène/méthanol 98:2) du résidu sec obtenu au stade précédent, en solution dans un mélange chlorure de méthylène-méthanol (50:50), a permis d'isoler en outre un composé C, de couleur violette (Rf=0,2).

L'analyse par CCM (éluant : mélange chlorure de méthylène/heptane 85:15) du résidu sec obtenu au stade précédent, en solution dans un mélange chlorure de méthylène-méthanol (50:50 en volume), a permis également d'isoler un composé D, de couleur jaune (Rf=0,14).

Les composés A, B, C et D ont été caractérisés par spectrométrie de masse, en RMN¹H, en RMN¹³C, en RMN 2D¹H-¹³C et ¹H-¹⁵N.

L'ensemble des données analytiques permet de proposer les structures suivantes :

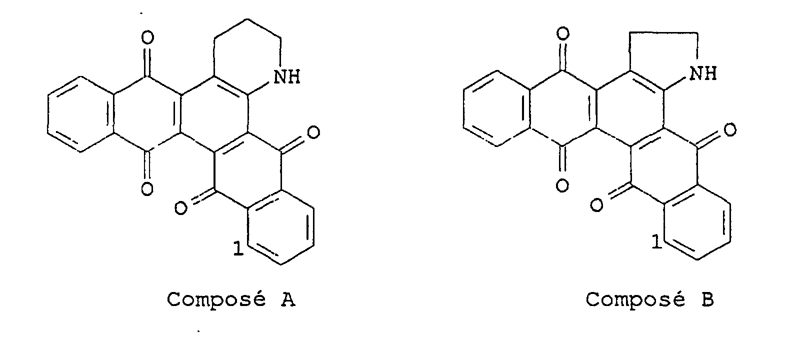

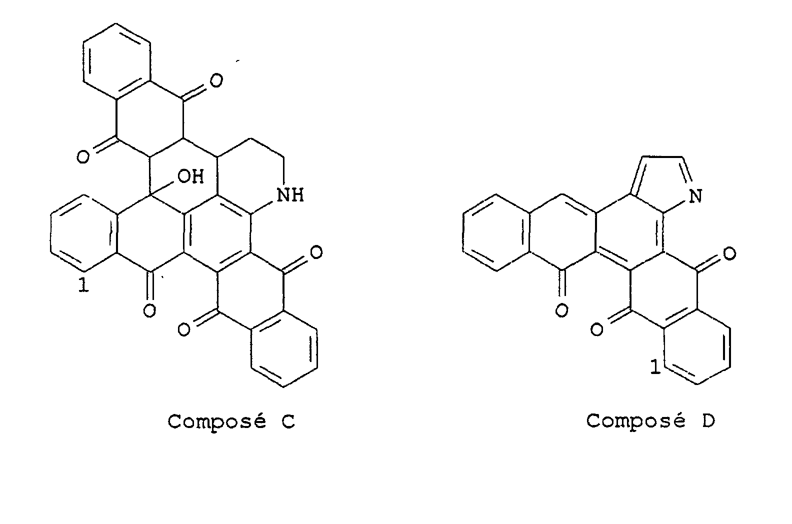

Le composé A est donc la 6,7,8,9-tétrahydro-dinaphto[2,3-f:2',3'-h] quinoléine-5,10,11,16-tétraone, le composé B est la dinaphto[2,3-e:2',3'-g]indoline-5,9,14,15-tétraone, le composé C est la 4b-hydroxy-4b,4c,10a,10b,11,12,13-heptahydro-3-aza-anthra[1,4-def]naptho[2',3'-p]chrysène-5,10,14,19,20-pentaone, et le composé D est la dinaptho[2,3-e:2',3'-g]indole-5,14,15-trione.

En solution dans le chlorure de méthylène, les composés A, B et C ont une couleur rouge et le composé D une couleur jaune.

Dans d'autres expériences, le filtrat obtenu à l'exemple 2a) ci-dessus a été traité à l'autoclave pendant 30 minutes à 120°C. Après addition de naphtoquinone et incubation comme ci-dessus, il n'y a pas formation des composés A et B. Par contre, un traitement du filtrat pendant 30 minutes à 80°C n'empêche pas l'obtention des produits A et B.

Lorsque le filtrat obtenu comme précédemment est préalablement traité par une protéase à une concentration finale de 80 unités pendant 15 heures à 40°C, on observe la formation des produits A et B, en quantité détectable, après réaction avec la 1,4-naphtoquinone dans les conditions déjà décrites ci-dessus.

Dans d'autres expériences, le filtrat obtenu à l'exemple 2a) ci-dessus a été dialysé à travers différentes membranes ayant des seuils de coupure respectifs de 3500, 1000 et 500 daltons. Dans chaque cas, la réaction avec la 1,4-naphtoquinone, suivie de l'extraction, comme décrit ci-dessus, a été effectuée à la fois sur le rétentat et sur le dialysat, et on a analysé par CCM les produits obtenus, en recherchant les produits A et B.

Avec les membranes ayant un seuil de coupure de 3500 et de 1000 daltons, les produits A et B sont détectés dans le produit obtenu par réaction avec le dialysat. Avec la membrane ayant un seuil de coupure de 500 daltons, les produits A et B se trouvent dans le produit obtenu par réaction avec le rétentat.

On peut donc conclure que les précurseurs de colorant, présents dans le filtrat, qui réagissent avec la 1,4-naphtoquinone, ont une masse moléculaire comprise entre 500 et 1000 daltons, et que la réaction avec la 1,4-naphtoquinone n'est pas de nature enzymatique.

### EXEMPLE 3 : Culture in vitro sous éclairemant : comparaison avec des cellules cultivées en présence de glucose

Lorsque la culture des cellules de luzerne, dans le même milieu qu'à l'exemple 1, est réalisée sous éclairement (photopériode de 16 heures), on obtient également la formation des produits A et B après réaction avec la 1,4-naphtoquinone comme décrit à l'exemple 2.

Divers essais analogues à ceux de l'exemple 2 ont été effectués sur des cultures de Medicago sativa cultivées sur le même milieu que celui décrit ci-dessus, mais en présence de glucose comme source de carbone au lieu de lactose. On n'a pas obtenu les produits A et B, en quantité détectable, après réaction avec la 1,4-naphtoquinone.

### EXEMPLE 4 : Réaction avec la p-benzoquinone

On opère de façon analogue à celle décrite à l'exemple 2b), en remplaçant la 1,4-naphtoquinone par la p-benzoquinone. Le mélange réactionnel obtenu a une coloration violette.

### EXEMPLE 5 : Réaction avec la juglone

On opère de façon analogue à celle décrite à l'exemple 2b), en remplaçant la 1,4-naphtoquinone par la 5-hydroxy 1,4-naphtoquinone (juglone). On obtient une coloration marron.

### EXEMPLE 6 : Réaction avec la naphtazarine

On opère de façon analogue à celle décrite à l'exemple 2b), en remplaçant la 1,4-naphtoquinone par la 5,8-dihydroxy 1,4-naphtoquinone (naphtazarine). On obtient une coloration rose.

### EXEMPLE 7 : Etude du pouvoir tinctorial

Les essais sont effectués sur des lots de cheveux contenant 90 % de cheveux blancs naturels.

Des essais ont également été effectués sur les mêmes cheveux préalablement permanentés avec la permanente DULCIA 1® (fournisseur : L'OREAL).

L'extrait obtenu à l'exemple 2c) ci-dessus est dissous dans l'éthanol à 95 %.

*Composition 1* : on ajoute à la solution éthanolique un égal volume d'eau additionnée d'acide citrique jusqu'à pH 4.

*Composition 2* : on ajoute à la solution éthanolique un égal volume d'eau additionnée de monoéthanolamine jusqu'à pH 10,5.

On immerge des mèches de cheveux de 1,2 g dans la composition 1 1 ou 2. On laisse agir pendant 30 minutes à 45°C, puis on rince les mèches et sèche les cheveux.

Dans tous les cas, on obtient une coloration tabac, plus marquée sur cheveux permanentés.

### EXEMPLE 8 : Composition de teinture pour cheveux

- Composé A 0,1 g
- Copolymère acétate de vinyle/acide crotonique/polyéthylène glycol vendu sous la dénomination "Aristoflex A" par la Société HOECHST 1,5 g
- Ethanol 40 g
- Triéthanolamine q.s.p. pH = 7
- Eau q.s.p. 100 g

Cette lotion est appliquée sur des cheveux blancs. Les cheveux sont alors mis en forme et séchés. Ils sont colorés en rouge.

On obtient des résultats analogues en remplaçant le composé A par le composé B ou C, ou par un mélange en quantités égales des composés A, B et C.

### EXEMPLE 9 : Composition de vernis à ongles

On prépare une composition pour coloration des ongles ayant la composition suivante (% en poids) :
- Toluène 21,97
- Acétate de butyle 10
- Acétate d'éthyle 10
- Acétate de n-propyle 10
- Isopropanol 25
- Nitrocellulose 9
- Phtalate de dibutyle 2
- Santolite 3
- Butyral polyvinylique 5
- Acétyl-tributyl-citrate 3
- Filtre U.V. 0,5
- Colorant 0,53

Lorsque le colorant est le composé A, B ou C, on obtient un vernis à ongles de couleur rose-rouge. Avec le composé D, la couleur du vernis est jaune.

### EXEMPLE 10 : Kit

La réaction de coloration peut être effectuée à l'aide d'un kit composé de 2 réactifs :
- un réactif A est constitué d'un flacon de verre de 10 mL contenant 50 µL d'une solution méthanolique de 1,4-naphtoquinone à 12,5 g/L,
- un réactif B est constitué d'un flacon de verre de 5 mL contenant 5 mL de réactif préparé comme suit : des cellules de luzerne (5 g) sont broyées dans un tampon comme décrit à l'exemple 1. Le milieu obtenu est filtré en conditions aseptiques sur membrane Millipore de 0,2 µ. 5 mL de ce mélange sont transférés de façon aseptique dans un flacon de verre préalablement stérilisé par autoclavage, 120°C, 30 minutes.

Ces deux réactifs peuvent donc se conserver ainsi à température ambiante durant quelques semaines.

Au moment de l'utilisation, le contenu du réactif B est transféré dans le flacon contenant le réactif A. Celui-ci est alors placé au bain-marie pour incubation pendant 90 minutes à 30°C, ce qui conduit à la formation des colorants recherchés.

## Revendications

1. Procédé de préparation d'une composition contenant un précurseur de colorants, caractérisé par le fait que l'on broie des plantes ou parties de plantes du genre Medicago, ou des cellules d'une plante du genre Medicago provenant d'une culture in vitro, et que l'on sépare et recueille selon les méthodes de fractionnement usuelles une fraction au moins partiellement purifiée exempte de constituants de masse moléculaire supérieure à 3500, ladite fraction étant capable de réagir en solution aqueuse avec la 1,4-naphtoquinone en donnant au moins un produit colorant.

2. Procédé selon la revendication 1, dans lequel, pour séparer ladite fraction au moins partiellement purifiée, on opère selon l'une au moins des méthodes suivantes :
- on procède à une filtration et recueille le filtrat ;
- on procède à une décantation ou à une centrifugation et on recueille le surnageant ;
- on procède à une extraction avec un solvant, puis, si désiré, on évapore ledit solvant.

3. Procédé selon la revendication 2 dans lequel, en outre, on traite un broyat, un extrait, un filtrat ou un surnageant ainsi obtenu de façon à éliminer les constituants de masse moléculaire supérieure à 1500.

4. Procédé selon l'une quelconque des revendications précédentes, caractérisé par le fait que l'on cultive *in vitro* des cellules d'une plante du genre Medicago, dans un milieu contenant une source de carbone appropriée, on broie la biomasse formée, et on sépare et recueille ladite fraction au moins partiellement purifiée.

5. Procédé selon la revendication précédente, caractérisé par le fait que l'on effectue ladite culture dans un milieu contenant uniquement du lactose comme source de carbone.

6. Procédé selon l'une quelconque des revendications 4 et 5, caractérisé par le fait que l'on effectue ladite culture à l'obscurité.

7. Composition, contenant au moins un précurseur de colorant, pouvant être obtenue selon le procédé de l'une quelconque des revendications précédentes.

8. Composition selon la revendication 7, caractérisée par le fait qu'elle se présente sous la forme d'une solution ou sous la forme d'un produit solide soluble dans un solvant choisi parmi l'eau, les alcanols en C₁-C₆, le chlorure de méthylène, et leurs mélanges.

9. Composition selon la revendication 7 ou 8, caractérisée par le fait que ledit précurseur est présent dans ladite composition à une concentration supérieure à la concentration dudit précurseur dans ladite plante ou dans lesdites cellules à l'état desséché.

10. Composition selon l'une quelconque des revendications 7 à 9, caractérisée par le fait que ledit précurseur de colorant est présent dans ladite composition à une concentration au moins égale à celle dudit précurseur dans une dispersion aqueuse contenant, par litre de dispersion, 1 gramme, en matière sèche, d'un broyât de cellules de ladite plante, les cellules dudit broyat ayant été cultivées *in vitro* dans un milieu contenant uniquement du lactose comme source de carbone.

11. Composition selon l'une quelconque des revendications 7 à 10, caractérisée par le fait que ladite plante est choisie parmi Medicago sativa, Medicago falcata, Medicago intermedia, Medicago media, Medicago varia et Medicago versicolor.

12. Composition selon l'une quelconque des revendications 7 à 11, caractérisée par le fait que ledit précurseur est capable de réagir avec la 1,4-naphtoquinone en donnant l'un au moins des produits suivants :
- 6,7,8,9-tétrahydro-dinaphto[2,3-f:2',3'-h]quinoléine 5,10,11,16-tétraone,
- dinaphto[2,3-e:2',3'-g]indoline-5, 9,14,15-tétraone,
- 4b-hydroxy-4b,4c,10a,10b,11,12,13-heptahydro-3-aza-anthra[1,4-def]naphto[2',3'-p]chrysène-5,10,14,19, 20-pentaone, et
- dinaphto [2,3-e:2',3'-g]indole-5,14,15-trione.

13. Procédé d'obtention d'un produit colorant, caractérisé par le fait que l'on fait réagir une quinone avec une composition telle que définie dans l'une quelconque des revendications 7 à 12, et que, si désiré, on isole et/ou purifie un produit colorant issu de cette réaction.

14. Procédé selon la revendication précédente, caractérisé par le fait que ladite quinone est choisie parmi les 1,4-benzoquinones éventuellement substituées en l'une au moins des positions 2 et 3, les 1,4-naphtoquinones éventuellement substituées en l'une au moins des positions 5, 6, 7 et 8, et la diphénoquinone.

15. Procédé selon la revendication précédente, caractérisée par le fait que ladite quinone est une 1,4-benzoquinone éventuellement substituée de formule (I) ou une 1,4-naphtoquinone éventuellement substituée de la formule (II): 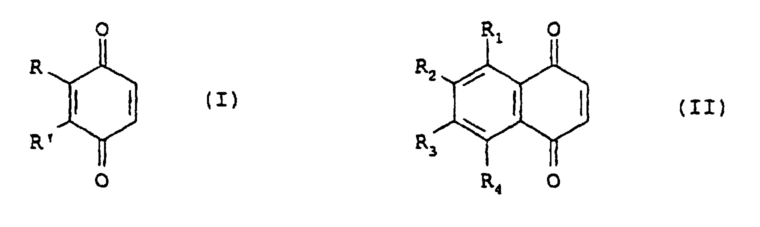 dans lesquelles les substituants *R,* R' et R₁ à R₄ représentent indépendamment -H, hydroxy, alkyle en C₁-C₄, alcoxy en C₁-C₄, sulfonyle, ou un groupement hétérocyclique ou aryle éventuellement substitué.

16. Procédé selon l'une quelconque des revendications 13 à 15, caractérisé par le fait que ladite quinone est choisie parmi la 1,4-benzoquinone, la 2-méthyl-1,4-benzoquinone, la 2-(4-méthyl-2-furanyl)-1,4-benzoquinone, la 2-hydroxy-1,4-benzoquinone, la 2-phényl-1,4-benzoquinone, la 1,4-naphtoquinone, la 5-hydroxy-1,4-naphtoquinone, la 5-hydroxy-7-méthyl-1,4-naphtoquinone, la 6-méthyl-1,4-naphtoquinone et la 5,8-dihydroxy-1,4-naphtoquinone.

17. Procédé selon l'une quelconque des revendications 13 à 16, caractérisé par le fait que l'on effectue ladite réaction en solution à une température pouvant aller de 20 à 100°C environ.

18. Produit colorant caractérisé par le fait qu'il est susceptible d'être obtenu par le procédé de l'une quelconque des revendications 13 à 17.

19. Produit colorant selon la revendication précédente, caractérisé par le fait qu'il est choisi parmi les suivants :
- 6,7,8,9-tétrahydro-dinaphto[2,3-f:2',3'-h] quinoléine 5,10,11,16- tétraone,
- dinaphto[2,3-e:2',3'-g]indoline-5,9,14,15-tétraone,
- 4b-hydroxy-4b,4c,10a,10b,11,12,13-heptahydro-3-aza-anthra[1,4-def]naphto[2',3'-p]chrysène-5,10,14,19,20-pentaone, et
- dinaphto[2,3-e:2',3'-g]indole-5,14,15-trione.

20. Kit permettant de préparer une composition colorante, ledit kit comprenant, dans des conteneurs séparés dont les contenus sont à mélanger au moment de l'emploi, au moins un précurseur de colorant et au moins une quinone, ledit précurseur présentant les caractéristiques suivantes :
- c'est un produit existant à l'état naturel dans les cellules d'une plante du genre Medicago,
- il est capable de réagir avec ladite quinone en donnant au moins un produit colorant.

21. Kit selon la revendication 20, caractérisé par le fait que ladite plante est choisie parmi celles mentionnées dans la revendication 11.

22. Kit selon la revendication 20 ou 21, caractérisé par le fait que ladite quinone est choisie parmi celles mentionnées dans l'une quelconque des revendications 14 à 16.

23. Composition colorante, caractérisée par le fait qu'elle contient un produit colorant tel que défini dans l'une quelconque des revendications 18 et 19.

## Patentansprüche

1. Verfahren zur Herstellung einer Zubereitung, welche eine Farbstoffvorstufe enthält, **dadurch gekennzeichnet**, dass man Pflanzen oder Teile von Pflanzen der Gattung Medicago oder Zellen einer Pflanze der Gattung Medicago, entstammend einer *in vitro*-Kultur, zerkleinert und dass man gemäß üblicher Fraktionierungsverfahren eine zumindest teilweise gereinigte Fraktion, die frei ist von Bestandteilen mit einem Molekulargewicht oberhalb von 3500, abtrennt und sammelt, wobei die Fraktion in wässriger Lösung mit 1,4-Naphthochinon reagieren kann, um mindestens ein Farbstoffprodukt zu ergeben.

2. Verfahren nach Anspruch 1, in dem zur Abtrennung der zumindest teilweise gereinigten Fraktion nach zumindest einem der folgenden Verfahren gearbeitet wird:
- Durchführung einer Filtration und Auffangen des Filtrats;
- Dekantieren oder Zentrifugieren und Sammeln des Überstands;
- Durchführung einer Extraktion mit einem Lösungsmittel, dann, falls gewünscht, Verdampfen des Lösungsmittels.

3. Verfahren nach Anspruch 2, in dem außerdem eine so erhaltene zerkleinerte Masse, einen erhaltenen Extrakt, ein erhaltenes Filtrat oder einen erhaltenen Überstand so behandelt, dass die Bestandteile mit einem Molekulargewicht oberhalb 1500 entfernt werden.

4. Verfahren nach einem der vorstehenden Ansprüche, **dadurch gekennzeichnet**, dass Zellen einer Pflanze der Gattung Medicago *in vitro* in einem Medium kultiviert werden, das eine geeignete Kohlenstoffquelle enthält, die gebildete Biomasse zerkleinert wird und die zumindest teilweise gereinigte Fraktion abgetrennt und gesammelt wird.

5. Verfahren nach dem vorstehenden Anspruch, **dadurch gekennzeichnet**, dass die Kultur in einem Medium durchgeführt wird, das nur Lactose als Kohlenstoffquelle enthält.

6. Verfahren nach einem der Ansprüche 4 und 5, **dadurch gekennzeichnet**, dass die Kultur in der Dunkelheit durchgeführt wird.

7. Zubereitung, enthaltend mindestens eine Farbstoffvorstufe, welche gemäß des Verfahrens nach einem der vorstehenden Ansprüche erhalten werden kann.

8. Zubereitung nach Anspruch 7, **dadurch gekennzeichnet**, dass diese in Form einer Lösung oder in Form eines festen Produkts vorliegt, welches löslich ist in einem Lösungsmittel, ausgewählt unter Wasser, C₁-C₆-Alkanolen, Methylenchlorid und Gemischen davon.

9. Zubereitung nach Anspruch 7 oder 8, **dadurch gekennzeichnet**, dass die Vorstufe in der Zubereitung in einer Konzentration oberhalb der Konzentration der Vorstufe in der Pflanze oder in den Zellen in getrocknetem Zustand vorliegt.

10. Zubereitung nach einem der Ansprüche 7 bis 9, **dadurch gekennzeichnet**, dass die Farbstoffvorstufe in der Zubereitung in einer Konzentration vorliegt, die mindestens derjenigen der Vorstufe in einer wässrigen Dispersion entspricht, welche pro Liter der Dispersion 1 g an Trockenmasse einer zerkleinerten Zellmasse der Pflanze enthält, wobei die Zellen der zerkleinerten Masse *in vitro* in einem Medium kultiviert wurden, welches nur Lactose als Kohlenstoffquelle enthält.

11. Zubereitung nach einem der Ansprüche 7 bis 10, **dadurch gekennzeichnet**, dass die Pflanze ausgewählt wird unter Medicago sativa, Medicago falcata, Medicago intermedia, Medicago media, Medicago varia und Medicago versicolor.

12. Zubereitung nach einem der Ansprüche 7 bis 11, **dadurch gekennzeichnet**, dass die Vorstufe in der Lage ist, mit 1,4-Naphthochinon zu reagieren, um mindestens eines der folgenden Produkte zu ergeben:
- 6,7,8,9-Tetrahydrodinaphtho[2,3-f:2',3'-h]chinolin-5,10,11,16-tetraon,
- Dinaphtho[2,3-e;2',3'-g]indolin-5,9,14,15-tetraon,
- 4b-Hydroxy-4b,4c,10a,10b,11,12,13-heptahydro-3-aza-anthra[1,4-def]-naphtho[2',3'-p]chrysen-5,10,14,19,20-pentaon, und
- Dinaphtho[2,3-e:2',3'-g]indol-5,14,15-trion.

13. Verfahren zur Darstellung eines Farbstoffprodukts, **dadurch gekennzeichnet**, dass ein Chinon mit einer Zubereitung, wie sie in einem der Ansprüche 7 bis 12 definiert ist, umgesetzt wird, und dass, falls gewünscht, ein aus dieser Reaktion entstandenes Farbstoffprodukt isoliert und/oder gereinigt wird.

14. Verfahren nach dem vorstehenden Anspruch, **dadurch gekennzeichnet**, dass das Chinon unter den 1,4-Benzochinonen, die gegebenenfalls in zumindest einer der Stellungen 2 und 3 substituiert sind, unter den 1,4-Naphthochinonen, die gegebenenfalls zumindest in einer der Stellungen 5, 6, 7 und 8 substituiert sind, und dem Diphenochinon ausgewählt wird.

15. Verfahren nach dem vorstehenden Anspruch, **dadurch gekennzeichnet**, dass das Chinon ein gegebenenfalls substituiertes 1,4-Benzochinon der Formel (I) oder ein gegebenenfalls substituiertes 1,4-Naphthochinon der Formel (II) ist: 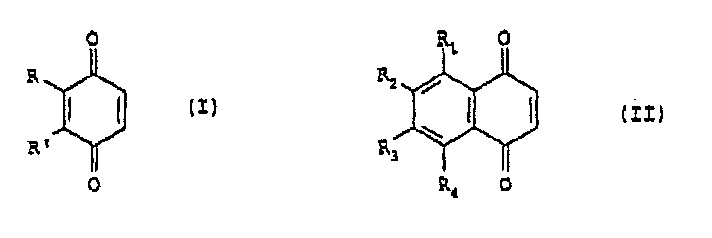 wobei die Substituenten R, R' und R₁ bis R₄ unabhängig voneinander -H, Hydroxy, C₁-C₄-Alkyl, C₁-C₄-Alkoxy, Sulfonyl oder eine gegebenenfalls substituierte heterocyclische oder Arylgruppe darstellen.

16. Verfahren nach einem der Ansprüche 13 bis 15, **dadurch gekennzeichnet**, dass das Chinon ausgewählt wird unter 1,4-Benzochinon, 2-Methyl-1,4-benzochinon, 2-(4-Methyl-2-furanyl)-1,4-benzochinon, 2-Hydroxy-1,4-benzochinon, 2-Phenyl-1,4-benzochinon, 1,4-Naphthochinon, 5-Hydroxy-1,4-naphthochinon, 5-Hydroxy-7-methyl-1,4-naphthochinon, 6-Methyl-1,4-naphthochinon und 5,8-Dihydroxy-1,4-naphthochinon.

17. Verfahren nach einem der Ansprüche 13 bis 16, **dadurch gekennzeichnet**, dass die Reaktion in Lösung bei einer Temperatur, die ungefähr von 20 bis 100 °C gehen kann, durchgeführt wird.

18. Farbstoffprodukt, **dadurch gekennzeichnet**, dass dieses durch das Verfahren nach einem der Ansprüche 13 bis 17 erhalten werden kann.

19. Farbstoffprodukt nach dem vorstehenden Anspruch, **dadurch gekennzeichnet**, dass dieses ausgewählt wird unter den folgenden:
- 6,7,8,9-Tetrahydrodinaphtho[2,3-f:2',3'-h]-chinolin-5,10,11,16-tetraon,
- Dinaphtho[2,3-e:2',3'-g]indolin-5,9,14,15-tetraon,
- 4b-Hydroxy-4b,4c,10a,10b,11,12,13-heptahydro-3-aza-anthra[1,4-def]-naphtho[2',3'-p]chrysen-5,10,14,19,20-pentaon, und
- Dinaphtho[2,3-e:2',3'-g]indol-5,14,15-trion.

20. Ausstattung zur Herstellung einer Farbstoffzubereitung, die Ausstattung umfassend, in getrennten Behältern, deren Inhalte im Moment der Verwendung gemischt werden, mindestens eine Farbstoffvorstufe und mindestens ein Chinon, wobei die Vorstufe die folgenden Eigenschaften aufweist:
- Es handelt sich um ein Produkt, das in natürlichem Zustand in den Zellen einer Pflanze der Gattung Medicago vorkommt,
- Sie kann mit dem Chinon reagieren, um mindestens ein Farbstoffprodukt zu ergeben.

21. Ausstattung nach Anspruch 20, **dadurch gekennzeichnet**, dass die Pflanze unter denjenigen ausgewählt wird, welche in Anspruch 11 erwähnt werden.

22. Ausstattung nach Anspruch 20 oder 21, **dadurch gekennzeichnet**, dass das Chinon unter denjenigen ausgewählt wird, welche in einem der Ansprüche 14 bis 16 erwähnt werden,

23. Farbstoffzubereitung, **dadurch gekennzeichnet**, dass diese ein Farbstoffprodukt enthält, welches in einem der Ansprüche 18 und 19 definiert ist.

## Claims

1. Process for preparing a composition containing a dye precursor, **characterized in that** plants, or parts of plants, of the Medicago genus, or cells from a plant of the Medicago genus originating from an in vitro culture, are ground, and in that an at least partially purified fraction free of constituents of molecular mass greater than 3 500 are separated and recovered according to conventional fractionation methods, said fraction being capable of reacting in aqueous solution with 1,4-naphthoquinone, giving at least one dye product.

2. Process according to Claim 1, in which, in order to separate said at least partially purified fraction, at least one of the following methods is carried out:
- filtration is carried out and the filtrate is recovered;
- decanting or centrifugation is carried out and the supernatant is recovered;
- extraction is carried out with a solvent and then, if desired, said solvent is evaporated off.

3. Process according to Claim 2, in which, in addition, ground material, an extract, a filtrate or a supernatant thus obtained is treated so as to eliminate the constituents of molecular mass greater than 1 500.

4. Process according to any one of the preceding claims, **characterized in that** cells of a plant of the Medicago genus are cultured *in vitro,* in a medium containing a suitable carbon source, the biomass formed is ground, and said at least partially purified fraction is separated and recovered.

5. Process according to the preceding claim, **characterized in that** said culturing is carried out in a medium containing only lactose as the carbon source.

6. Process according to either of Claims 4 and 5, **characterized in that** said culturing is carried out in the dark.

7. Composition, containing at least one dye precursor, which can be obtained according to the process of any one of the preceding claims.

8. Composition according to Claim 7, **characterized in that** it is in the form of a solution or in the form of a solid product which is soluble in a solvent chosen from water, C₁-C₆ alkanols, methylene chloride and mixtures thereof.

9. Composition according to Claim 7 or 8, **characterized in that** said precursor is present in said composition at a concentration greater than the concentration of said precursor in said plant or in said cells in the desiccated state.

10. Composition according to any one of Claims 7 to 9, **characterized in that** said dye precursor is present in said composition at a concentration at least equal to that of said precursor in an aqueous dispersion containing, per litre of dispersion, 1 gram, in solids, of ground material from cells of said plant, the cells of said ground material having been cultured *in vitro* in a medium containing only lactose as the carbon source.

11. Composition according to any one of Claims 7 to 10, **characterized in that** said plant is chosen from Medicago sativa, Medicago falcata, Medicago intermedia, Medicago media, Medicago varia and Medicago versicolor.

12. Composition according to any one of Claims 7 to 11, **characterized in that** said precursor is capable of reacting with 1,4-naphthoquinone, giving at least one of the following products:
- 6,7,8,9-tetrahydrodinaphtho[2,3-f:2',3'-h]-quinoline-5,10,11,16-tetraone,
- dinaphtho[2,3-e:2',3'-g]indoline-5,9,14,15-tetraone,
- 4b-hydroxy-4b,4c,10a,10b,11,12,13-heptahydro-3-aza-anthra[1,4-def]naphtho[2',3'-p]chrysene-5,10,14,19,20-pentaone, and
- dinaphtho[2,3-e:2',3'-g]indole-5,14,15-trione.

13. Process for producing a dye product, **characterized in that** a quinone is reacted with a composition as defined in any one of Claims 7 to 12, and in that, if desired, a dye product derived from this reaction is isolated and/or purified.

14. Process according to the preceding claim, **characterized in that** said quinone is chosen from 1,4-benzoquinones optionally substituted at at least one of positions 2 and 3, 1,4-naphthoquinones optionally substituted at at least one of positions 5, 6, 7 and 8, and diphenoquinone.

15. Process according to the preceding claim, **characterized in that** said quinone is an optionally substituted 1,4-benzoquinone of formula (I) or an optionally substituted 1,4-napthoquinone of formula (II): 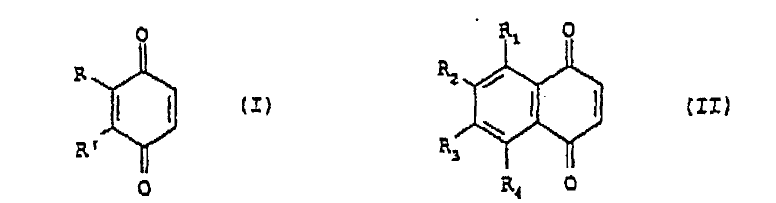 in which the substituents R, R' and R₁ to R₄ represent, independently, -H, hydroxy, C₁-C₄ alkyl, C₁-C₄ alkoxy, or sulphonyl, or an optionally substituted heterocyclic or aryl group.

16. Process according to any one of Claims 13 to 15, **characterized in that** said quinone is chosen from 1,4-benzoquinone, 2-methyl-1,4-benzoquinone, 2-(4-methyl-2-furanyl)-1,4-benzoquinone, 2-hydroxy1,4-benzoquinone, 2-phenyl-1,4-benzoquinone, 1,4-naphthoquinone, 5-hydroxy-1,4-naphthoquinone, 5-hydroxy-7-methyl-1,4-naphthoquinone, 6-methyl-1,4-naphthoquinone and 5,8-dihydroxy-1,4-naphthoquinone.

17. Process according to any one of Claims 13 to 16, **characterized in that** said reaction is carried out in solution at a temperature which can range from 20 to 100°C approximately.

18. Dye product, **characterized in that** it can be obtained using the process of any one of Claims 13 to 17.

19. Dye product according to the preceding claim, **characterized in that** it is chosen from the following:
- 6,7,8,9-tetrahydrodinaphtho[2,3-f:2',3'-h]quinoline-5,10,11,16-tetraone,
- dinaphtho[2,3-e:2',3'-g]indoline-5,9,14,15-tetraone,
- 4b-hydroxy-4b,4c,10a,10b,11,12,13-heptahydro-3-aza-anthra[1,4-def]naphtho[2',3'-p]chrysene-5,10,14,19,20-pentaone, and
- dinaphtho[2,3-e:2',3'-g]indole-5,14,15-trione.

20. Kit for preparing a dye composition, said kit comprising, in separate containers, the contents of which are to be mixed at the time of use, at least one dye precursor and at least one quinone, said precursor having the following characteristics:
- it is a product which exists in the natural state in the cells of a plant of the Medicago genus,
- it is capable of reacting with said quinone, giving at least one dye product.

21. Kit according to Claim 20, **characterized in that** said plant is chosen from those mentioned in Claim 11.

22. Kit according to Claim 20 or 21, **characterized in that** said quinone is chosen from those mentioned in any one of Claims 14 to 16.

23. Dye composition, **characterized in that** it contains a dye product as defined in either of Claims 18 and 19.
